(19) Europäisches Patentamt
European Patent Office
Office européen des brevets

(11) Numéro de publication : **0 279 716 B1**

(12) # FASCICULE DE BREVET EUROPEEN

(45) Date de publication du fascicule du brevet : **17.04.91 Bulletin 91/16**

(51) Int. Cl.[5] : **C07C 231/02**, C07C 233/31, C07C 233/36

(21) Numéro de dépôt : **88400088.6**

(22) Date de dépôt : **18.01.88**

(54) **Procédé de n-omega-trifluoroacétylation des alpha,omega diaminoacides monocarboxyliques aliphatiques saturés.**

Le dossier contient des informations techniques présentées postérieurement au dépôt de la demande et ne figurant pas dans le présent fascicule.

(30) Priorité : **26.01.87 FR 8701031**

(43) Date de publication de la demande : **24.08.88 Bulletin 88/34**

(45) Mention de la délivrance du brevet : **17.04.91 Bulletin 91/16**

(84) Etats contractants désignés : **AT BE CH DE ES FR GB GR IT LI LU NL SE**

(56) Documents cités :
**CHEMICAL ABSTRACTS, vol. 106, no. 1, 5 janvier 1987, pages 651-652, résumé no. 19027t, Columbus, Ohio, US; T.J. BLACKLOCK et al.: "Large scale N-carboxyanhydride preparation of Ala-Pro and N-(Tfa)-Lys-Pro: synthesis of ACE inhibitors", & PEPT.: STRUCT. FUNCT. PROC. AM. PEPT. SYMP., 9th 1985, 787-90**

(56) Documents cités :
**JOURNAL OF THE AMERICAN CHEMICAL SOCIETY, vol. 77, no. 10, 1 juin 1955, pages 2779-2783, American Chemical Society; H.B. JONASSEN et al.: "The reaction between the copper(II)-ethylenediamine complexes and erythritol anhydride"**
**THE JOURNAL OF ORGANIC CHEMISTRY, vol. 44, no. 15, 20 juillet 1979, pages 2805-2807, American Chemical Society; T.J. CURPHEY: "Trifluoroacetylation of amino acids and peptides by ethyl trifluoroacetate"**

(73) Titulaire : **RHONE-POULENC CHIMIE**
**25, quai Paul Doumer**
**F-92408 Courbevoie Cédex (FR)**

(72) Inventeur : **Aviron-Violet, Paul**
**25bis, chemin de la Citadelle**
**F-69230 Saint Genis Laval (FR)**
Inventeur : **Gervais, Christian**
**6, allée Achard**
**F-69100 Villeurbanne (FR)**

(74) Mandataire : **Le Pennec, Magali et al**
**RHONE-POULENC INTERSERVICES Service Brevets Chimie 25, Quai Paul Doumer**
**F-92408 Courbevoie Cédex (FR)**

**EP 0 279 716 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de N-s trifluoracétylation des α, ω diaminoacides monocarboxyliques aliphatiques saturés.

Il est connu de trifluoroacétyler les amino-acides basiques, tels que la lysine ou l'ornithine... par l'anhydride trifluoroacétique en milieu acide F. Weygand Chem. Be. 89, 647 (1956) ; il a été constaté que la réaction de trifluoroacétylation des acides aminés présentant deux fonctions amines a lieu préférentiellement sur la fonction amine en α du groupe carboxyle ou sur les deux fonctions amines simultanément.

Il a été proposé E. Schallenberg et M. Calvin, Journal of American Chem. Soc. 77, 2779, (1955) de trifluoroacétyler la fonction amine en bout de chaine des acides aminés tels que lysine et ornithine, par réaction avec le trifluoroacétate de thio-éthanol en milieu basique aqueux. Outre le fait que le trifloroacétate de thioéthanol est un produit cher, polluant et conférant une inévitable odeur soufrée à l'acide aminée préparé, ce procédé ne permet pas de N-trifluoroacétyler en ω les α, ω diaminoacides monocarboxyliques aliphatiques saturés avec une sélectivité suffisante. Ce procédé, en effet, n'empêche pas la formation de dérivés α trifluoroacétylés et α, ω ditrifluoroacétylés en quantité notable. T. J. Curphey J. Org. Chem., 44 (15) 2805 (1979) a décrit la N-trifluoroacétylation d'α amino-acides ne présentant qu'une seule fonction amine, à l'aide de trifluoroacétate d'éthyle en présence d'une base de type triéthylamine et de méthanol anhydre pendant au moins quatorze heures. Les produits obtenus sont N-α trifluoroacétylés. La demanderesse a constaté que ce procédé ne permet pas de N-trifluoroacétyler régiosélectivement en ω, les α, ω diaminoacides monocarboxyliques aliphatiquement saturés.

La demanderesse a trouvé un procédé permettant de N-trifluoroacétyler régiosélectivement en ω les α-ω diaminoacides monocarboxyliques aliphatiques.

La présente invention concerne un procédé de N-ω trifluoroacétylation des α, ω-diaminoacides monocarboxyliques aliphatiques saturés à l'aide d'un trifuloroacétate d'alkyle linéaire ou ramifié en $C_1$-$C_4$, caractérisé en ce que la réaction est menée dans un agent de dilution choisi parmi les alconols en $C_1$-$C_4$ linéaire ou ramifié et ou les trifluoroacétates d'alkyle linéaire ou ramifié en $C_1$-$C_4$.

Les α, ω diamino acides monocarboxyliques aliphatiques saturés mis en oeuvre sont racémiques ou optiquement actifs et peuvent être représentés par la formule suivante :

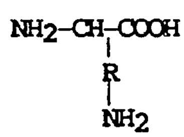

où R représente un radical alkylène en $C_1$-$C_6$ linéaire ou ramifié.

Ces composés peuvent être avantageusement la lysine ou l'ornithine.

L'opération de N-ω trifluoroacétylation peuvent être réalisée en mettant en oeuvre de 1 à 2 moles de trifluoroacétate d'alkyle, de préference de 1 à 1,25 moles, par mole d'α, ω diamino acide monocarboxylique aliphatique saturé.

L'agent de dilution peut être constitué par un excès du trifluoroacétate d'alkyle.

Ledit alconol peut être utilisé pur ou bien peut contenir jusqu'à 10% de son volume d'eau, de préférence 5%. Ledit excès de trifluoroacétate d'alkyle peut également contenir jusqu'à 10% de son volume d'eau, mais il est préférable de l'utiliser pur.

La quantité d'agent de dilution mise en oeuvre peut aller de 0,5 et 10 litres, de préférence de 0,5 et 2 litres, par mole de N-a,s diamino acide monocarboxylique aliphatique saturé, de façon à obtenir un milieu d'agitation facile.

Parmi les alconols pouvant être mis en oeuvre, on peut citer : le méthanol, l'éthanol, l'isopropanol et le tertio butanol.

L'opération de N-ω trifluoroacétylation peut être favorablement réalisée à pression atmosphérique, à des températures de l'ordre de 0 à 50°C, de préférence de 20 à 25°C.

L'opération de N-ω trifluoroacétylation dure également de l'ordre de 15 minutes à 15 heures, préférentiellement de 15 minutes à 3 heures.

Le produit N-ω trifluoroacétylé obtenu est ensuite séparé du milieu réactionnel, par exemple par tout procédé physique de séparation telle que la filtration, et il peut être ensuite purifié par tout moyen connu, par exemple par recristallisation dans un mélange eau-alcool. (Ces opérations peuvent être répétées plusieurs fois si nécessaire).

Le procédé faisant l'objet de l'invention présente l'avantage de ne nécessiter la présence d'aucun réactif

autre que l'α, ω diaminoacide monocarboxylique aliphatique saturé, le trifluoroacétate d'alkyle et un agent de dilution ; notamment la présence d'une trialkylamine n'est pas nécessaire et elle a même plutôt tendance à nuire à la bonne marche de la réaction de N-ω trifluoroacétylation.

Ce procédé permet d'obtenir les dérivés N-ω trifluoroacétylés des α, ω diamino acides carboxyliques aliphatiques saturés avec une sélectivité en dérivé N-ω trifluoroacétylé, supérieure à 90% par rapport à la quantité d'α, ω diamino acide carboxylique aliphatique saturé transformé.

Les dérivés N-ω trifluoroacétylés obtenus selon le procédé de l'invention peuvent être utilisés notamment pour la préparation de dipeptides carboxyalkylés, par exemple selon le procédé décrit dans la demande de brevet européen N° 168 769.

Les exemples suivants illustrent l'invention sans toutefois la limiter.

Example 1 :

Dans un réacteur de 3 litres, on charge 1,61 moles (235 g) de L lysine et 1,61 litres d'éthanol.

On agite ce mélange au moyen d'un barreau magnétique, et on y ajoute en une demi-heure, 2,02 moles (287 g) de trifluoroacétate d'éthyle.

Le mélange est laissé sous agitation pendant une heure et demie.

On récupère par filtration 337 g d'un précipité que l'on sèche.

Ce précipité est analysé par chromatographie liquide haute performance. On constate qu'il titre :

– 88% en poids de N-ε trifluoroacétate de lysine,

– 12% en poids de lysine non transformée (on ne trouve que des traces d'α trifluoroacétate d'éthyle et d'α, ε ditrifluoroacétate d'éthyle),

ce qui correspond à un rendement en ε trifluoroacétate de lysine brut de 76%.

On recristallise 280 g de ce précipité dans 3,76 l d'éthanol à reflux, auquel on ajoute peu à peu 1,12 l d'eau jusqu'à dissolution complète.

Après refroidissement, l'ε trifluoroacétate de lysine précipite, la lysine restant en solution aqueuse.

L'ε trifluoroacétate de lysine précipité est filtré puis séché.

On recueille alors 170 g d'ε trifluoroacétate de lysine, dont le titre est voisin de 100% et dont le pouvoir rotatoire est : $\alpha_D^{20}$ = +20,6° (c = 3 acide dichloroacétique) [le pouvoir rotatoire théorique de ce produit est : $\alpha_D^{20}$ = +20,5°], ce qui correspond à un rendement en ε trifluoroacétate de lysine cristallisé de 52,5%.

Par concentration du filtrat et recristallisation, on récupère encore 52 g d'ε trifluoroacétate de lysine.

On obtient donc un rendement global en N-ε trifluoroacétate de lysine recristallisée de 68,5%, par rapport à la lysine de départ.

La structure de l'ε trifluoroacétate de lysine est confirmée par spectrométrie de masse ou RMN.

Exemple 2 :

On reproduit dans cet exemple, l'essai décrit dans l'exemple n°1, mais en remplaçant les 1,61 litres d'éthanol par un même volume de trifluoroacétate d'éthyle.

Le précipité obtenu à la fin de la période d'agitation (1 heure et demie), est lavé avec de l'éthanol jusqu'à élimination de toute trace de trifluoroacétate d'éthyle ; on obtient les rendements suivants, en produits bruts :

ε trifluoroacétate de lysine : 90%

α trifluoroacétate de lysine : 1%

α,ε ditrifluoroacétate de lysine : traces

La sélectivité relative en ε trifluoroacétate de lysine est de : 98,9%.

Exemples 3 à 16

Ces exemples ont été réalisés selon le mode opératoire de l'exemple 1.

La nature des réactifs mis en oeuvre, leur quantité ainsi que les conditions opératoires figurent au tableau I.

Les rendements en ε trifluoroacétate de lysine brut, α, ε ditrifluoroacétate de lysine brut et α trifluoroacétate de lysine brut, figurant également au tableau I, montrent que la sélectivité de l'ε trifluoroacétate de lysine est supérieure à 90%.

Exemple 17

On reproduit dans cet exemple, l'essai décrit dans l'exemple 14, mais en remplaçant les 5 m-moles de trifluoroacétate d'éthyle par 5 m-moles de trifluoroacétate de méthyle.

A la fin de la période d'agitation (2 heures), on obtient les rendements en produits bruts suivants :

ε trifluoroacétate de lysine : 79%

α trifluoroacétate de lysine : 1,5%

α, ε ditrifluoroacétate de lysine : traces

La sélectivité relative en ε trifluoroacétate de lysine est de 98%.

Exemples 18 à 21 :

Ces exemples donnés à titre comparatif sont réalisés selon le mode opératoire des exemples 3 à 16, mais en présence d'une base selon le procédé décrit par Curphey [J. Org. Chem., 44, (15), 2805 (1979)].

La nature des réactifs mis en oeuvre, leur quantité ainsi que les conditions opératoires figurent au tableau II.

On constate que la sélectivité en ε trifluoroacétate de lysine est bien inférieure à 90%.

Exemples 22 à 25 :

Ces exemples sont donnés à titre comparatif.

L'agent trifluoroacétylant employé ici, est le trifluoroacétate de thioéthanol des les conditions décrites par E. Schallenberg et M. Calvin [Journal of American Chem. Soc. 77, 2779, (1955)].

Ces exemples sont réalisés de la manière suivante :

on charge 10,3 moles de lysine dans un réacteur de 50 ml ; on introduit 21 ml de NaOH 0,5 N (qui dissout la lysine en quelques minutes) sous agitation. Le pH est alors entre 10,6 et 11 ; puis on ajuste le pH à température ambiante à la valeur désirée avec de l'acide sulfurique 9 N ou 3 N ; on coule alors en 20 minutes le trifluoroacétate de thioéthanol ; puis on ajuste le pH par coulée de soude N.

Les résultats obtenus à différents pH, figurent au tableau III.

La sélectivité en ε trifluoroacétate de lysine est toujours largement inférieure à 90%.

Tableau I

| Ex. | Lysine | Et TFA | Alcanol | | t°C | durée | Rendements en % en TFA (lys) brut | | | Sélectivité relative en ε TFA en % |
|---|---|---|---|---|---|---|---|---|---|---|
| | m-mole | m-mole | nature | ml | | heure | ε | α,ε | α | |
| 3 | 5 | 5 | Et OH | 5 | 25 | 2 | 40 | traces | traces | # 100 |
| 4 | 5 | 6,25 | Et OH | 5 | 25 | 2 | 84 | traces | traces | # 100 |
| 5 | 5 | 7,5 | Et OH | 5 | 25 | 2 | 85 | traces | traces | # 100 |
| 6 | 5 | 6,25 | Et OH | 5 | 25 | 1/4 | 63 | traces | traces | # 100 |
| 7 | 5 | 6,25 | Et OH | 5 | 25 | 1/2 | 76 | traces | traces | # 100 |
| 8 | 5 | 6,25 | Et OH | 5 | 25 | 1 | 74 | traces | traces | # 100 |
| 9 | 5 | 6,25 | Et OH | 5 | 25 | 3 | 76 | traces | traces | # 100 |
| 10 | 5 | 6,25 | Et OH | 5 | 50 | 2 | 32 | traces | traces | # 100 |
| 11 | 5 | 6,25 | Et OH | 10 | 25 | 2 | 80 | traces | traces | # 100 |
| 12 | 5 | 6,25 | Me OH | 2,5 | 25 | 3 | 76 | traces | traces | # 100 |
| 13 | 5 | 6,25 | Me OH | 3,5 | 25 | 3 | 80 | traces | traces | # 100 |
| 14 | 5 | 6,25 | Me OH | 5 | 25 | 2 | 78 | 3,5 | traces | 95,7 |
| 15 | 5 | 6,25 | Isopropanol | 5 | 25 | 1H3/4 | 84 | traces | traces | # 100 |
| 16 | 5 | 6,25 | Tertiobutanol | 5 | 25 | 1H3/4 | 68 | traces | traces | # 100 |

Et TFA : trifluoroacétate d'éthyle
ε TFA (lys) : ε trifluoroacétate de lysine
α,εTFA (lys) :α,εditrifluoroacétate de lysine
α TFA (lys) : α trifluoroacétate de lysine

Et OH : éthanol
Me OH : méthanol

Tableau II

| Ex. | Lysine | Et TFA | Alcanol | | Triéthylamine | t°C | durée | Rendements en % en TFA(lys) brut | | | Sélectivité relative en TFA en % |
|---|---|---|---|---|---|---|---|---|---|---|---|
| | m-mole | m-mole | nature | ml | m-mole | | heure | ε | α, ε | α | |
| 18 | 5 | 6,25 | Me OH | 2,5 | 5 | 25 | 15 | 58 | 23 | 3 | 69 |
| 19 | 5 | 6,25 | Me OH | 2,5 | 10 | 25 | 15 | 45 | 25 | 4 | 60,8 |
| 20 | 5 | 6,25 | Me OH | 2,5 | 5 | 50 | 15 | 62 | 13,5 | 3 | 79 |
| 21 | 5 | 6,25 | Me OH | 2,5 | 5 | 25 | 3 | 50 | 35 | 10 | 52,6 |

Tableau III

| Ex. | Lysine | Et SH TFA | pH | t°C | durée | Sélectivité relative en % en TFA (lys) brut | | |
|---|---|---|---|---|---|---|---|---|
| | m-mole | m-mole | | | heure | ε | α,ε | α |
| 22 | 10,3 | 16,4 | 7,5 | 25 | 16 | 55 | 17 | 28 |
| 23 | 10,3 | 16,4 | 8 | 25 | 21 | 43,5 | 25,5 | 31 |
| 24 | 10,3 | 16,4 | 9 | 25 | 17 | 34 | 13 | 5 |
| 25 | 10,3 | 16,4 | 10 | 25 | 1H10 | 85,5 | 12 | 2,5 |

Et SH TFA : trifluoroacétate de thioéthanol

**Revendications**

1. Procédé de N ω trifluoroacétylation des α,ω-diaminoacides monocarboxyliques aliphatiques saturés à l'aide d'un trifluoroacétate d'alkyle linéaire ou ramifié en $C_1$-$C_4$, caractérisé en ce que la réaction est menée dans un agent de dilution choisi parmi les alconols en $C_1$-$C_4$ linéaire ou ramifié et/ou les trifluoroacétates d'alkyle linéaire ou ramifié en $C_1$-$C_4$.

2. Procédé selon la revendication 1 caractérisé par le fait que α,ω-diamino-monocarboxylique mise en oeuvre sont représentés par la formule :

$$NH_2-CH-COOH$$
$$!$$
$$R$$
$$!$$
$$NH_2$$

où R représente un radical alkylène en $C_1$-$C_6$ linéaire ou ramifié.

3. Procédé selon l'une quelconque des revendications précédentes caractérisé en ce que l'α,ω-diaminoacide monocarboxylique est la lysine ou l'ornithine.

4. Procédé selon l'une quelconque des revendications précédentes caractérisé en ce que l'opération de N ω trifluoroacétylation est réalisé à l'aide de 1 à 2 mole de trifluoroacétate d'alkyle par mole d'α, ω diaminoacide monocarboxylique aliphatique saturé.

5. Procédé selon la revendication 4 caractérisé en ce que l'opération de N ω trifluoroacétylation est réalisée à l'aide de 1 à 1,25 moles de trifluoroacétate d'alkyle par mole d'α, ω diaminoacide monocarboxylique saturé.

6. Procédé selon l'une des revendications 1 à 5 caractérisé en ce qu'il n'y a de réactif autre que α, ω diaminoacide monocarboxylique aliphatique saturé, le trifluoroacétate d'alkyle et un agent de dilution.

7. Procédé selon l'une des revendications 1 à 5 caractérisé en ce qu'il a lieu sans présence d'une trialkylamine.

8. Procédé selon l'une des revendications 1 à 7 caractérisé en ce que l'agent de dilution est constitué par de l'éthanol, de l'isopropanol ou du tertiobutanol.

9. Procédé selon l'une des revendications de 1 à 8 précédentes caractérisé en ce que l'agent de dilution contient jusqu'à 10% de son volume d'eau.

10. Procédé selon l'une des revendications 1 à 9 caractérisé en ce que le volume d'agent de dilution utilisé est compris entre 0,5 et 10 litres par mole de N α, ω diaminoacide monocarboxylique aliphatique saturé.

11. Procédé selon la revendication 10 caractérisé en ce que le volume d'agent de dilution utilisé est compris entre 0,5 et 2 litres par mole de N α, ω diaminoacide monocarboxylique aliphatique saturé.

12. Procédé selon l'une quelconque des revendications précédentes caractérisé en ce que l'opération de N ω trifluoroacétylation est réalisée à pression atmosphérique, à une température comprise entre 0 et 50°C.

13. Procédé selon la revendication 12 caractérisé en ce que l'opération de N ω trifluoroacétylation est réalisée à des températures comprises entre 20 et 25°C.

14. Procédé selon l'une quelconque des revendications précédentes caractérisé en ce que l'opération de N ω trifluoroacétylation est réalisée sous agitation pendant 15 minutes à 15 heures.

15. Procédé selon la revendication 14 caractérisé en ce que l'opération de N ω trifluoroacétylation est réalisée pendant 2 à 3 heures.

16. Procédé de préparation du N ω trifluoroacétate de lyse par N-ω trifluoroacétylation de la lysine par le trifluoroacétate d'éthyle ou de méthyle en présence d'éthanol.

17. Produits obtenus par le procédé faisant l'objet de l'une quelconque des revendications précédentes caractérisé en ce qu'ils présentent une régiosélectivité en ω supérieure à 90%.

**Ansprüche**

1. Verfahren zur N-ω-Trifluoracetylierung der gesättigten aliphatischen α,ω-Diamino-monocarbonsäuren mit Hilfe eines Alkyltrifluoracetats mit linearer oder verzweigter $C_1$-$_4$-Alkylgruppe, dadurch gekennzeichnet, daß

die Reaktion in einem Verdünnungsmittel durchgeführt wird, das unter den linearen oder verzweigten $C_1$-$C_4$-Alkanolen und/oder den Alkyltrifluoracetaten mit linearer oder verzweigter $C_1$-$C_4$-Alkylgruppe ausgewählt ist.

2. Verfahren nach Anspruch 1, dadurch gekennzeichnet, daß die eingesetzten α,ω-Diamino-monocarbonsäuren durch die Formel

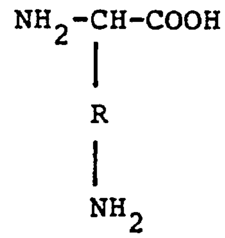

wiedergegeben werden, in der R eine lineare oder verzweigte $C_1$-$C_6$-Alkylengruppe bedeutet.

3. Verfahren nach einem der vorangehenden Ansprüche, dadurch gekennzeichnet, daß die α,ω-Diamino-monocarbonsäure Lysin oder Ornithin ist.

4. Verfahren nach einem der vorangehenden Ansprüche, dadurch gekennzeichnet, daß die N-ω-Trifluoracetylierung mit Hilfe von 1 bis 2 mol Alkyltrifluoracetat je mol gesättigte aliphatische α,ω-Diamino-monocarbonsäure durchgeführt wird.

5. Verfahren nach Anspruch 4, dadurch gekennzeichnet, daß die N-ω-Trifluoracetylierung mit Hilfe von 1 bis 1,25 mol Alkyltrifluoracetat je mol gesättigte α,ω-Diamino-monocarbonsäure durchgeführt wird.

6. Verfahren nach einem der Ansprüche 1 bis 5, dadurch gekennzeichnet, daß kein anderer Reaktionspartner vorhanden ist als gesättigte aliphatische α, ω-Diamino-monocarbonsäure, Alkyltrifluoracetat und Verdünnungsmittel.

7. Verfahren nach einem der Ansprüche 1 bis 5, dadurch gekennzeichnet, daß es ohne Anwesenheit eines Trialkylamins durchgeführt wird.

8. Verfahren nach einem der Ansprüche 1 bis 7, dadurch gekennzeichnet, daß das Verdünnungsmittel Ethanol, Isopropanol oder tert.-Butanol ist.

9. Verfahren nach einem der vorangehenden Ansprüche 1 bis 8, dadurch gekennzeichnet, daß das Verdünnungsmittel bis zu 10% seines Volumens Wasser enthält.

10. Verfahren nach einem der Ansprüche 1 bis 9, dadurch gekennzeichnet, daß das eingesetzte Volumen Verdünnungsmittel 0,5 bis 10 l je mol gesättigte aliphatische N-α,ω-Diamino-monocarbonsäure ausmacht.

11. Verfahren nach Anspruch 10, dadurch gekennzeichnet, daß das Volumen des eingesetzten Verdünnungsmittels 0,5 bis 2 l je mol gesättigte aliphatische N-α,ω-Diamino-monocarbonsäure ausmacht.

12. Verfahren nach einem der vorangehenden Ansprüche, dadurch gekennzeichnet, daß die N-ω-Trifluoracetylierung unter Atmosphärendruck bei einer Temperatur im Bereich von 0 bis 50°C durchgeführt wird.

13. Verfahren nach Anspruch 12, dadurch gekennzeichnet, daß die N-ω-Trifluoracetylierung bei Temperaturen im Bereich von 20 bis 25°C durchgeführt wird.

14. Verfahren nach einem der vorangehenden Ansprüche, dadurch gekennzeichnet, daß die N-ω-Trifluoracetylierung unter Rühren während 15 min bis zu 15 h durchgeführt wird.

15. Verfahren nach Anspruch 14, dadurch gekennzeichnet, daß die N-ω-Trifluoracetylierung während 2 bis 3 h durchgeführt wird.

16. Verfahren zur Herstellung von Lysin-N-ω-trifluoracetat durch N-ω-Trifluoracetylierung von Lysin mit Ethyl- oder Methyltrifluoracetat in Gegenwart von Ethanol.

17. Produkte, erhalten durch das Verfahren nach einem der vorangehenden Ansprüche, dadurch gekennzeichnet, daß sie eine Regioselektivität für ω von mehr als 90% aufweisen.

## Claims

1. Process for the N-ω-trifluoroacetylation of saturated aliphatic monocarboxylic α,ω-diamino acids using a $C_1$-$C_4$ straight-chain or branched alkyl trifluoroacetate characterized in that the reaction is conducted in a diluting agent chosen from $C_1$-$C_4$ straight-chain or branched alkanols and/or $C_1$-$C_4$ straight-chain or branched alkyl trifluoroacetates.

2. Process according to Claim 1, characterized in that the monocarboxylic α,ω-diamino acids used are represented by the formula :

$$NH_2-CH-COOH$$
$$|$$
$$R$$
$$|$$
$$NH_2$$

where R represents a $C_1$-$C_6$ straight-chain or branched alkylene radical.

3. Process according to either of the preceding claims, characterized in that the monocarboxylic α,ω-diamino acid is lysine or ornithine.

4. Process according to any one of the preceding claims, characterized in that the N-ω-trifluoroacetylation operation is carried out using 1 to 2 moles of alkyl trifluoroacetate per mole of saturated aliphatic monocarboxylic α,ω-diamino acid.

5. Process according to Claim 4, characterized in that the N-ω-trifluoroacetylation operation is carried out using 1 to 1.25 moles of alkyl trifluoroacetate per mole of saturated monocarboxylic α,ω-diamino acid.

6. Process according to one of Claims 1 to 5, characterized in that there is no reagent other than saturated aliphatic monocarboxylic α,ω-diamino acid, alkyl trifluoroacetate and a diluting agent.

7. Process according to one of Claims 1 to 5, characterized in that it takes place without the presence of a trialkylamine.

8. Process according to one of Claims 1 to 7, characterized in that the diluting agent consists of ethanol, isopropanol or tert-butanol.

9. Process according to one of Claims from 1 to 8 above, characterized in that the diluting agent contains up to 10% of its volume of water.

10. Process according to one of Claims 1 to 9, characterized in that the volume of diluting agent employed is between 0.5 and 10 litres per mole of saturated aliphatic monocarboxylic N-α,ω-diamino acid.

11. Process according to Claim 10, characterized in that the volume of diluting agent employed is between 0.5 and 2 litres per mole of saturated aliphatic monocarboxylic N-α,ω-diamino acid.

12. Process according to any one of the preceding claims, characterized in that the N-ω-trifluoroacetylation operation is carried out at atmospheric pressure at a temperature of between 0 and 50°C.

13. Process according to Claim 12, characterized in that the N-ω-trifluoroacetylation operation is carried out at temperatures of between 20 and 25°C.

14. Process according to any one of the preceding claims, characterized in that the N-ω-trifluoroacetylation operation is carried out with stirring for 15 minutes to 15 hours.

15. Process according to Claim 14, characterized in that the N-ω-trifluoroacetylation operation is carried out over 2 to 3 hours.

16. Process for the preparation of lysine N-ω-trifluoroacetate by the N-ω-trifluoroacetylation of lysine with ethyl trifluoroacetate or methyl trifluoroacetate in the presence of ethanol.

17. Products obtained by the process forming the subject of any one of the preceding claims, characterized in that they have an ω-regioselectivity greater than 90%.